# EUROPEAN PATENT APPLICATION

(11) **EP 4 653 038 A1**
(43) Date of publication of application: **26.11.2025**
(21) Application number: 24305807.0
(22) Date of filing: 23.05.2024
(51) Int. Cl.: A61M 25/01, A61M 25/09

(54) **ELONGATED STEERABLE DEVICE WITH DETERMINED CURVATURE**

(71) Applicant: Basecamp Vascular, 51100 Reims (FR)
(72) Inventor: CAZENEUVE, Jean-Baptiste, 92190 MEUDON (FR)
(74) Representative: Icosa

(57) **Abstract**

The invention relates to an elongated steerable device for guiding a catheter or an endoscope comprising an elongated flexible member, at least one shape memory alloy wire (16) presenting at least one activable zone, and an actuator configured to activate the at least one shape memory alloy wire,
wherein the activable zone (17) of the at least one shape memory alloy wire (16) is configured to present a resting configuration and an activated configuration, the activated configuration displaying at least one predetermined curvature (C1, C2),
wherein the activable zone (17) of the at least one shape memory alloy wire (16) is configured to adopt the activated configuration when activated by the actuator, thus inducing the predetermined curvature (C1, C2) to the distal extremity (12D) of the elongated flexible member (12).

## Description

### FIELD OF INVENTION

The present invention relates to an elongated steering system configured to guide a catheter or an endoscope for internally investigating a tubular element, which presents at least one actuatable curvature.

### BACKGROUND OF INVENTION

There is a wide range of applications where there is a need for using a device inside a pipe, a duct, or a tube, for example for the placement of the distal part of a flexible tube in a specific location, for investigation or bringing pharmaceuticals, or functionality at a remote or hard-to-access location.

When displacing a flexible elongated device in the lumen of a pipe, duct or a tube, it is important for the user to be able to control carefully and precisely the movement and the placement of such device. Placement of such devices within pipes is a known technical issue in oil engineering or in motor engineering. Placement of devices within body tubes, such as for example through the ostium (of a vein, an artery, the gastrointestinal track...etc.), is also known in the medical field as being challenging.

In the medical field, using surgical or endovascular techniques, one can treat a lot of cardiovascular diseases causing death in the world. One of the pathologies encountered is the myocardial infarction along with peripheral vascular diseases. Through the last decades, the use of catheters and guidewires to reach pathological areas so as to deliver stents or balloons has emerged as an easy-to-implement solution. These endovascular techniques are less invasive compared to conventional surgeries. They come with a decreased recovery time and less post operation complications.

However, generally speaking, the surgeon skill and experience are a major success factor in the complex interventions while recent developments aim at facilitating navigation through complex anatomies as independently as possible from those surgeon skill and experience.

The devices currently known from the state of the art, are either complex and/or cumbersome and do not offer enough bending ability to navigate inside the lumen of a tube or pipe.

Usual shape memory alloy steering devices are composed of a shape memory alloy wire crimped on both extremities to the structure they are configured to deflect. With these devices, it is by design, hard to obtain high deflection angles with a tight radius of curvature due to the low contraction rate of the shape memory alloy wires (below 8%). Such a wide curvature radius renders the device difficult to use in very tortuous conducts.

Further to the rather wide curvature radius, the classical devices are difficult to assemble as the wire needs to be precisely and firmly maintained at each of its extremities to the structure to be bent/deflected. It also leads to devices which are quick to be worn out and are not functional on a very long term.

The main objective of the present invention is to provide a steerable elongated device which is easy to handle and to produce, compact and repeatable, and presents a curvature angle comprised between 0 and 360°, preferably ranging from 90 and 180°. According to the invention, the steerable device obtained is flexible enough to allow easy navigation inside a lumen, tube or pipe and strong enough to drive a catheter or an endoscope. The high curvature angle of the steerable elongated device should enable to access places, in the human anatomy, which are difficult to reach for. This way, the present invention enables to avoid the use of a well-known "exchange guide" during intervention.

### SUMMARY

This invention thus relates to an elongated steerable device for guiding a catheter or an endoscope comprising:
- an elongated flexible member extending along a longitudinal axis, the flexible member having a proximal extremity and a distal extremity,
- at least one shape memory alloy wire presenting at least one activable zone and secured to the distal extremity of the elongated flexible member,
- an actuator configured to activate the at least one shape memory alloy wire from the proximal extremity of the elongated flexible member,

**wherein** the activable zone of the at least one shape memory alloy wire is configured to present a resting configuration and an activated configuration, the activated configuration displaying at least one predetermined curvature,
**wherein** the activable zone of the at least one shape memory alloy wire is configured to adopt the activated configuration when activated by the actuator, thus inducing the predetermined curvature to the distal extremity of the elongated flexible member.

Thus, this solution achieves the above objective. In particular, it allows the obtaining of improved performances and easiness of manufacturing compared to shape memory alloy based steering systems that use contraction of a shape memory alloy actuator. As any curvature angle can be reached, the mobility with regards of the steering devices of the state of the art is necessarily improved.

The device according to the invention may comprises one or several of the following features, taken separately from each other or combined with each other:
- the actuator comprises heating means associated to the at least one shape memory alloy wire, those heating means being configured to heat said at least one shape memory alloy wire in a controlled manner in order to bring the alloy wire in its activated configuration,
- the elongated flexible member comprises a super-elastic base,
- the elongated flexible member comprises a metallic base,
- the diameter of the distal extremity of the elongated flexible member is inferior to the diameter of the proximal extremity of the elongated flexible member,
- the device further comprises an elastic element configured to maintain the at least one shape memory alloy wire in a predetermined resting shape when said at least one shape memory alloy wire is in its resting configuration,
- the elastic element is an elastic straightening element configured to maintain the at least one shape memory alloy wire in a straight shape when said at least one shape memory alloy wire is in its resting configuration,
- the at least one shape memory alloy wire presents a diameter of at least 180µm,
- the at least one shape memory alloy wire presents a diameter of a least 200µm,
- the at least one shape memory alloy wire presents a variable diameter along the longitudinal axis,
- the at least one shape memory alloy wire is secured in a U shape to the to the distal extremity of the elongated flexible member,
- the activable zone of the at least one shape memory alloy wire displays at least two predetermined curvatures when put in its activated configuration,
- the at least one shape memory alloy wire comprises at least two active zones which can be activated independently from each other and wherein the actuator is configured to activate each active zone independently from each other,
- the securing sheath displays heat isolation properties.

### BRIEF DESCRIPTION OF THE DRAWINGS

The invention will be better understood, and other aims, details, characteristics and advantages thereof will emerge more clearly on reading the detailed explanatory description which follows, of embodiments of the invention given by way of illustration. purely illustrative and non-limiting examples, with reference to the accompanying drawings:
- **figure 1** is a side view of the device according to the present invention in a resting configuration and an activated configuration,
- **figure 2** is a partial perspective view of a first embodiment of the device according to the present invention in its activated configuration,
- **figure 3** is a partial perspective view of a second embodiment of the device according to the present invention in its activated configuration,
- **figure 4** is a perspective longitudinal section of the second embodiment of a device according to the present invention,
- **figure 5** is a detailed perspective view of the wire in its resting configuration,
- **figure 6** is a partial perspective view of a third embodiment of the device according to the present invention in its activated configuration.

### DETAILED DESCRIPTION

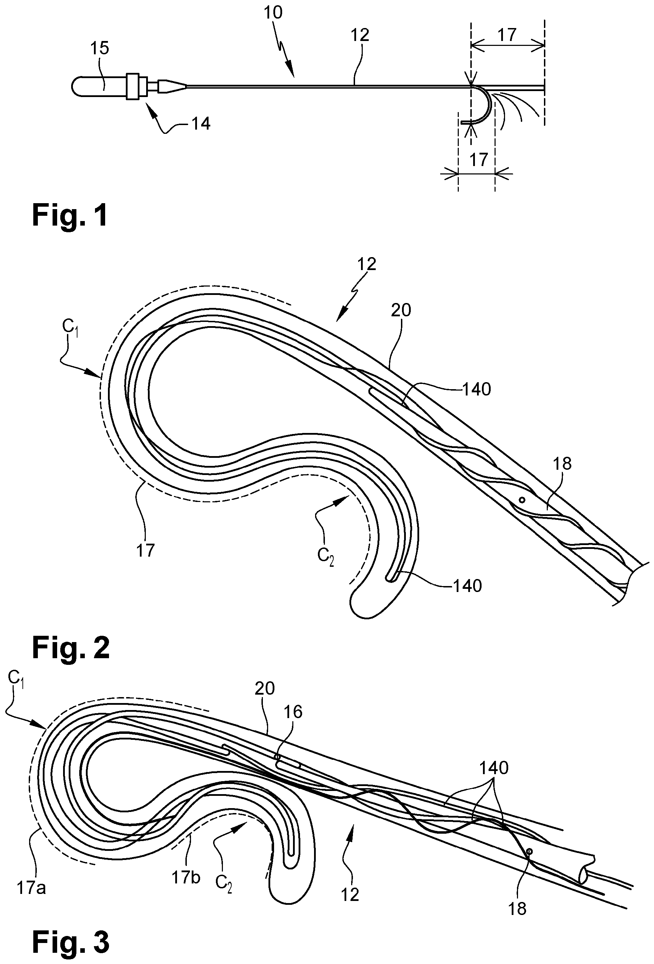

As can be seen on figure 1, this invention relates to an elongated steerable device 10 for guiding a catheter or an endoscope inside a tubular cavity, for example during a surgical intervention.

The elongated steerable device 10 comprises:
- an elongated flexible member 12 extending along a longitudinal axis X, the flexible member 12 having a proximal extremity 12P and a distal extremity 12D,
- an actuator 14 comprising at least one actuation means 140 controlled by a handle 15, the handle 15 being secured to the proximal extremity 12P of the elongated flexible element 12 (see figure 1),
- at least one shape memory alloy wire 16 with at least one activable zone 17.

The at least one shape memory alloy wire 16 forms at least partially the distal extremity 12D of the flexible member 12 (see figures 2 to 6). The distal extremity 12D of the flexible member 12 further comprises the activable zone 17 of the shape memory alloy wire 16.

The elongated flexible member 12 of the device 10 is discardable.

As can be seen on figures 3 to 6, in some embodiments, the shape memory alloy wire 16 presents a global U shape, the bottom of the U forming the end of the flexible member 12. This U shape of the shape memory alloy wire 16 enables to reinforce the strength of the shape memory alloy wire 16 by doubling its width while needing the same amount of energy to activate its activable zone 17 (see description below).

In order to ensure its structure and flexibility, the flexible member 12 comprises an elastic element 18 extending from its proximal extremity 12P towards its distal extremity 12D along the longitudinal axis X. The elastic element 18 does not necessarily extends up to the distal extremity 12D of the flexible member. In some alternative embodiments, the elastic element 18 extends over the whole flexible member 12 from its proximal extremity 12P to its distal extremity 12D along the longitudinal axis X. This elastic element 18 might for example be a hyper-elastic/super-elastic wire or tab connected to the shape memory alloy wire 16. In another embodiment, the elastic element 18 might be a hyper-elastic/super-elastic conduct surrounding the shape memory alloy wire 16.

In the present invention the term "hyper-elastic" refers to a model for expressing the stress-strain behaviour of some particular materials. For many materials, linear elastic models do not accurately describe the observed material behaviour. The most common example of this kind of material is rubber, whose stress-strain relationship can be defined as non-linearly elastic, isotropic and incompressible. Hyperelasticity provides a means of modeling the stress-strain behavior of such materials.

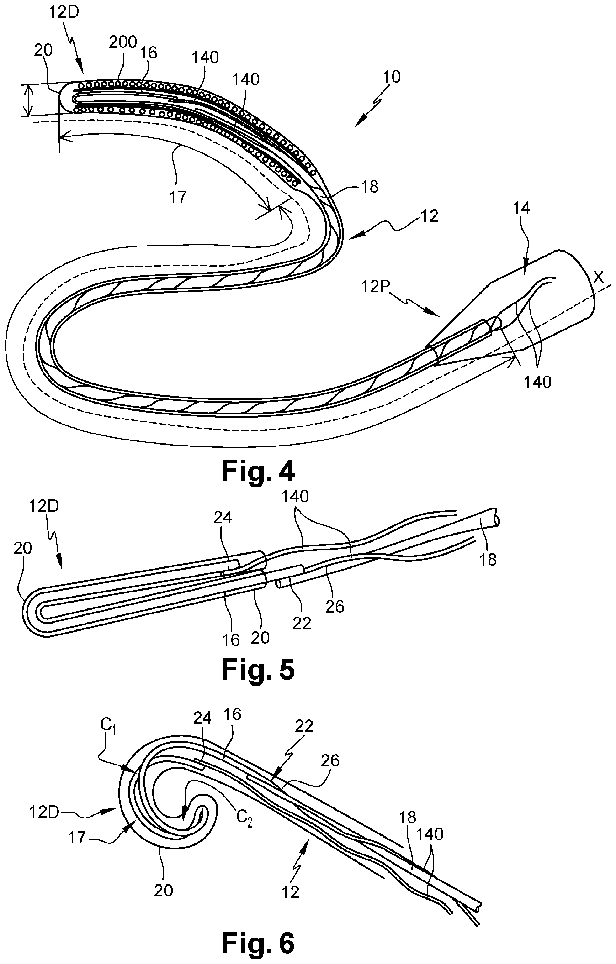

The connection 22 of the shape memory alloy wire 16 to the elastic element 18 enables to achieve a strong mechanical junction. This allows the transmission of torque from the proximal extremity 12P to the activable zone 17 inside the distal extremity 12D of the flexible member 12. This connection 22 can be achiever for example by welding. Preferably, the shape memory alloy wire 16 is connected to the elastic element 18 at one of its extremities, the other extremity being a free extremity (see figures 2 and 5).

Preferably, the elastic element 18 presents a variable diameter. Its diameter is larger at the proximal extremity 12P of the flexible member 12 and smaller at the distal extremity 12D of the flexible member 12. The diameter of the elastic element 18 ranges from 0,7 mm at the proximal extremity 12P of the flexible member 12 to 0,1mm at the distal extremity 12D of the flexible member 12. The diameter preferably ranges from 0,46 to 0,15mm. Thie diameter reduction enables an easier activation of the activable zone 17.

In order to activate the shape memory alloy wire 16 (see detailed explanations below), the device 10 comprises at least one actuation means 140 an energy source to the shape memory alloy wire 16.

The distal extremity 12D of the flexible member 12 further comprises the at least one actuation means 140. Preferably, the flexible member 12 comprises at least two actuation means 140 and, in some embodiments, the flexible member 12 comprises even three or more actuation means (see figure 3). Each actuation means 140 is preferably connected to the shape memory alloy wire 16. This connection can be a direct connection 24 or an indirect connection 26, through the elastic element 18. Each actuation means 140 is preferably connected to one extremity of the shape memory alloy wire 16 (see figures 2 and 4). In case there are more than two actuation means 140, the remaining actuation means are connected to the shape memory alloy wire 16 between its extremities (see figure 3). Each actuation means 140 can also be connected to the elastic element 18 in order to be supported and maintained in place even when the activable zone 17 is activated. It is preferably connected to the shape memory alloy wire 16 and to the elastic element 18. The connection 26 between the at least one actuation means 140 and the elastic element 18 can be achieved by welding. The connection 26 between the at least one actuation means 140 and the elastic element 18 is preferably located close or in direct contact with the connection 22 of the shape memory alloy wire 16 to the elastic element 18 (see figure 5).

The term "connected" in this embodiment is to be understand in a physical way and could be interpreted as "secured".

The at least one actuation means 140 extends from the proximal extremity 12P to the distal extremity 12D of the flexible member 12. The at least one actuation means 140 might be wrapped or coiled around the elastic element 18 along the length of the flexible member 12. A more detailed description of an embodiment will be presented further below. The functioning of the activation means will be detailed further below.

The flexible member 12 of the device 10 may also comprise a security shaft 20 configured to enclose the flexible member 12. This security shaft 20 is preferably made of plastic, for example from Pebax^{®}. In some embodiments, like the embodiment presented in figure 1, the security shaft 20 might present an evolving structure along the longitudinal axis X: on the proximal extremity 12P of the flexible member 12, it might comprise a single plastic layer while it may, on the distal extremity 12D of the flexible member 12 comprise a coil or spring 200 and several layers of plastic including Pebax^{®} and PTFE (Polytetrafluoroethylene) all layered in order to homogeneously surround the distal extremity 12D of the flexible member 12. The spring 200 might also help the distal extremity 12D to regain its resting configuration and ensures some straightness and some extremity rigidity in order to avoid unnecessary friction along the tubular cavity (which can measure over 1m50 length). This security shaft 20 is configured to isolate the environment in which the elongated steerable device 10 has to be introduced from, for example heat.

The elongated flexible member 12 can measure between 0,5m and 4m. The diameter of the elongated flexible member 12 can vary from 0,35mm at its distal extremity 12D to 2mm at its proximal extremity.

In metallurgy, a shape-memory alloy (SMA) is well known to be an alloy that can be deformed when cold ("cold configuration") but returns to its pre-deformed ("remembered configuration") shape when heated. It may also be called memory metal, memory alloy, smart metal, smart alloy, or muscle wire. In the present invention, the shape memory alloy wire 16 is made of such an alloy, for example Nitinol.

In the present invention, each shape memory alloy wire 16 is configured to present:
- a resting configuration corresponding to the "cold configuration" mentioned here-above, this resting configuration displaying a resting shape, and
- an activated configuration corresponding to the "remembered configuration" mentioned here-above, this activated configuration being a predetermined curved configuration different from the resting configuration (see figure 3).

In the present invention, the shape memory alloy wire 16 can present a "single shape memory" or a "double shape memory". In the case of a "single shape memory" the shape memory alloy wire 16 in its resting shape presents no particular shape and remains malleable. In this case the shape of the resting configuration is defined by its surroundings. In the case of a "double shape memory", the shape memory alloy wire 16 presents a memorized predetermined resting shape, for example a straight resting shape.

Depending on the embodiments, the shape memory alloy wire 16 presents a diameter comprised between 50 µm to 300 µm, preferably a diameter of at least 180µm. In some alternative embodiments, the shape memory alloy wire presents a diameter of a least 200µm. The diameter of the shape memory alloy wire 16 is important for mainly two reasons. Firstly, the shape memory alloy wire 16, once activated by the activation means 140 has to change its shape despite the resistance of the elongated flexible member 12 and the activation means 140 themselves which are made of material and therefore naturally exert some counter-force which can prevent the shape memory alloy wire 16 to reach its full deformation potential. Therefore, the bigger the shape memory alloy wire 16 is, the steadier and stronger the activated configuration is and thus enables a better handling of the device 10. On the other hand, the bigger a shape memory alloy wire 16 is, the more difficult it is to bring it back in its resting shape. This leads to an increased use of energy. As there is more material to deform, the provided activation energy (preferably heat) must be more consequent which can lead to isolation issues.

The advantages of a straight resting shape are to enable a facilitate way to navigate inside the arteries. Any remaining residual curvature, even a passive residual curvature, might generate frictions on the internal wall of the artery and this can restrict the navigation/steering performances.

In the present invention, the activable zone 17 of the shape memory alloy wire 16 is defined as the zone which has been pre-formed and is thus able to present a "cold configuration" and a "remembered configuration".

The distal extremity 12D thus corresponds to a controllable flexible and shape shifting portion of the elongated flexible member 12. This distal portion 12D of the elongated member 12 presents a length of about 10 to 20cm, preferably 15cm.

As previously mentioned, the actuator 14 is configured to activate the activable zone 17 of the at least one shape memory alloy wire 16 from the proximal extremity 12P of the elongated flexible member 12.

In order to activate the at least one activable zone 17 of the shape memory alloy wire 16, the actuator 14 comprises, as already mentioned, at least one activation means 140, preferably several activation means 140, associated to the shape memory alloy wire 16 and connected to the activable zone 17. The actuator 14 is also connected to a source of energy. The actuation means 140 are configure to convey the energy from the energy source towards the activable zone 17. The conveyed energy leads the activable zone 17 to be activated and to be deformed according to its activated configuration. The more energy is conveyed towards the activable zone 17, the closer it gets to its activated configuration and the longer it maintains it. Once the energy stopes being conveyed to the activable zone 17, it moves back in its resting configuration. The amount of the energy sent to the actuator 14 is preferably adjusted by means of the handle 15. Said handle 15 is designed to easily fit inside the hand of an operator. An operator can thus activate the activation means 140 by means of the handle 15 in order to lead the shape memory alloy wire 16 from its resting configuration to its activated configuration. The handle 15 further serves to push and steer the device 10 through the inside of the tubular cavity. The handle 15 may be removably connected to the elongated flexible member 12. The handle 15 may be either discardable along with the rest of the device 10 or may be reusable.

As can be seen on the embodiment depicted on figures 2 to 6, the elongated steerable device 10 comprises specific activation means 140 which are, in this embodiment, heating means 140. Those heating means 140 are associated to activable zone 17 of the shape memory alloy wire 16. The heating means 140 are connected to the handle 15 of the actuator 14 and enable to heat the activable zone 17 of the shape memory alloy wire 16 in a controlled manner. More specifically, as can be seen on figure 4, those heating means 140 are a first and a second cupper wires which each present a distal and a proximal extremity. The proximal extremities of the first and second copper wires are connected to the handle 15 of the actuator 14. The distal extremity of the first copper wire is directly connected to the free extremity of the shape memory alloy wire 16. The distal extremity of the second copper wire is indirectly connected to the other extremity of the shape memory alloy wire 16, through the elastic element 18. By activating the activation means 140 of the actuator 14 by means of the handle 15, current (thus heat) is generated and led along the elongated flexible member 12 towards the shape memory alloy wire 16, and more specifically towards the activable zone 17 of the shape memory alloy 16. In order to safely connect the shape memory alloy wire 16 with the handle 15 of the actuator 14 by means of the copper wires, said copper wires are wrapped around the elongated flexible member 12 between its proximal and distal extremities 12D, 12P.

In other embodiments, the activation means 140 could be any sort of conductive wire if the activation of the activable zone 17 is based on the Joule effect. In another embodiment, the activable zone 17 could be heated indirectly by means on a Kanthal wire. In a further embodiment, the activable zone 17 could be activated by means of a laser brought to the distal extremity of the flexible member 12 by means of an optical fiber. In this last example, the at least one activation means 140 is solely connected to the elastic element 18.

Depending on the embodiments and the needs, the activable zone 17, when activated, presents a curvature angle comprised between 0 and 360°, preferably ranging from 90 and 180°.

In some embodiments, the device 10 comprises several shape memory alloy wires 16, and each shape memory alloy wire 16 presents at least one activable zone 17 (not represented). Each shape memory alloy wire 16 presents the same activated configuration. All the shape memory alloy wires are activated by means of the same actuation means 140 and are deformed in a synchronic way. This enables to improve the strength of the distal extremity 12D of the elongated member 12.

In some alternative embodiment, regardless of the number of memory alloy wire 16, each memory alloy wire presents two distinct activable zones 17a, 17b (see figure 3). Each independent activable zone 17a, 17b is associated with independent activation means 140 (for example heating means) for activating (for example heating) each activable zone 17a, 17b in an independent controlled manner. Each activable zone 17a, 17b can present a different activated configuration.

When the activation means 140 of the actuator 14 are activated (for example through the handle 15), the energy (more particularly the heat) provided by the activation means 140 reaches the shape memory alloy wire 16, it changes its shape: it changes from its resting configuration (figure 4 and 5) to its activated configuration (see figures 1, 2, 3 and 6). This configuration change induces a shape change from the distal extremity 12D of the flexible member 12 from straight (resting) to curveted.

As can be seen on figures 2, 3 and 6, the shape memory alloy wire 16 induces, when activated, at least two predetermined curvatures C₁, C₂ to the distal extremity 12D of the elongated flexible member 12. Those multiple curvatures C₁, C₂ enable an easier introduction in particularly crooked and curved ducts.

This way, the shape memory alloy wire 16 induces, when activated, a predetermined curvature C₁, C₂, to the distal extremity 12D of the elongated flexible member 12. Preferably, the shape memory alloy wire 16 induces two different curvatures C₁, C₂ in order to achieve a more precise steering. The curvatures C₁, C₂ can present the same convexity (figures 2 and 3) or alternate convexities (figure 6).

In the embodiments comprising several activable zones 17a, 17b, each activable zone 17a, 17b preferably induces, when activated, a different predetermined curvatures C₁, C₂ to the distal extremity 12D of the elongated flexible member 12 (see figure 3). Alternatively, each shape memory alloy wire 16 can induces two different predetermined curvatures C₁, C₂ with only one activable zone 17 (see figure 2). Those two different predetermined curvatures C₁, C₂, in order to enable the formation of a specific scheme of pre-defined curvatures C₁, C₂ enabling a very specific and precise controlled shaping of the distal extremity 12D of the elongated member 12 of the device 10.

In the present invention, "to curvate": means to take the form of a curvature, to bend. Having a curvature or being curved is used in opposition to being straight. The term "curvature" means non-zero curvature. The curvature can be positive or negative.

In the particular embodiment depicted on figures 1 and 2, as already mentioned, the shape memory alloy wire 16 is secured in a U shape to the to the distal extremity 12D of the elongated flexible member 12. When shaped as a U, the shape memory alloy wire 16 presents two free extremities. Each free extremity of the U is oriented towards the proximal extremity 12P of the elongated flexible member 12 (see figure 2). This specific form firstly enables to easily connect the heating means 140 and the elastic element 18 to the extremities of the shape memory alloy wire 16 by a simple welding process which does not present risks of damaging the shape memory alloy wire 16. Secondly, this U shape enables to double the diameter of the effective shape memory alloy wire 16 as both branches of the U act the same in an almost perfect mirror effect (see figure 3). This leads to a stronger and steadier shaping of the distal extremality 12D of the flexible elongated member 12 without the necessity of providing and pre-shaping very thick and difficult to handle shape memory alloy wire 16. Thirdly, this U shape configuration enables to reduce the impact of the heating means 20 over the deformation of the shape memory alloy wire 16.

The device 10 of the present invention is really easy to manufacture as it allows an extremely simple assembly because the shape memory alloy wire can be connected/secured at only one of its extremities. No guidance along its entire length is necessary. Multiple curves on the same shape memory alloy 16 are also possible.

The precision of the device 10 is also increased as the shape taken by the activable zone 17 is known in advance, which leads to a more relatable tool.

## Claims

1. Elongated steerable device (10) for guiding a catheter or an endoscope comprising:
- an elongated flexible member (12) extending along a longitudinal axis (X), the flexible member (12) having a proximal extremity (12P) and a distal extremity (12D),
- at least one shape memory alloy wire (16) presenting at least one activable zone (17) and secured to the distal extremity (12D) of the elongated flexible member (12),
- an actuator (14) configured to activate the at least one shape memory alloy wire (16) from the proximal extremity (12P) of the elongated flexible member (12),
**wherein** the activable zone (17) of the at least one shape memory alloy wire (16) is configured to present a resting configuration and an activated configuration, the activated configuration displaying at least one predetermined curvature (C₁, C₂),
**wherein** the activable zone (17) of the at least one shape memory alloy wire (16) is configured to adopt the activated configuration when activated by the actuator (14), thus inducing the predetermined curvature (C₁, C₂) to the distal extremity (12D) of the elongated flexible member (12).

2. Elongated steerable device (10) according to the preceding claim, **wherein** the actuator (14) comprises heating means (20) associated to the at least one shape memory alloy wire (16), those heating means (20) being configured to heat said at least one shape memory alloy wire (16) in a controlled manner in order to bring the alloy wire (16) in its activated configuration.

3. Elongated steerable device (10) according to any one of the preceding claims, **wherein** the elongated flexible member (12) comprises a super-elastic base (22).

4. Elongated steerable device (10) according to any one of claims **1** to **3, wherein** the elongated flexible member (12) comprises a metallic base (22).

5. Elongated steerable device (10) according to any one of the preceding claims, **wherein** the diameter of the distal extremity (12D) of the elongated flexible member (22) is inferior to the diameter of the proximal extremity (12P) of the elongated flexible member (22).

6. Elongated steerable device (10) according to any one of the preceding claims, **wherein** the device (10) further comprises an elastic element (24) configured to maintain the at least one shape memory alloy wire (16) in a predetermined resting shape when said at least one shape memory alloy wire (16) is in its resting configuration.

7. Elongated steerable device (10) according to the preceding claim, **wherein** the elastic element (24) is an elastic straightening element configured to maintain the at least one shape memory alloy wire (16) in a straight shape when said at least one shape memory alloy wire (16) is in its resting configuration.

8. Elongated steerable device (10) according to any of the preceding claims, **wherein** the at least one shape memory alloy wire (16) presents a diameter of at least 180µm.

9. Elongated steerable device (10) according to the preceding claim, **wherein** the at least one shape memory alloy wire (16) presents a diameter of a least 200µm.

10. Elongated steerable device (10) according to any one of the preceding claims, **wherein** the at least one shape memory alloy wire (16) presents a variable diameter along the longitudinal axis (X).

11. Elongated steerable device (10) according to any one of the preceding claims, **wherein** the at least one shape memory alloy wire (16) is secured in a U shape to the to the distal extremity (12D) of the elongated flexible member (12).

12. Elongated steerable device (10) according to any one of the preceding claims, **wherein** the activable zone (17) of the at least one shape memory alloy wire (16) displays at least two predetermined curvatures (C₁, C₂) when put in its activated configuration.

13. Elongated steerable device (10) according to any one of the preceding claims, **wherein** the at least one shape memory alloy wire (16) comprises at least two active zones (17) which can be activated independently from each other and wherein the actuator (14) is configured to activate each active zone (17) independently from each other.

14. Elongated steerable device (10) according to any one of the preceding claims, **wherein** the securing sheath (18) displays heat isolation properties.
